# EUROPEAN PATENT SPECIFICATION

(11) **EP 0 966 431 B1**
(45) Date of publication and mention of the grant of the patent: **12.12.2001**
(21) Application number: 98916997.4
(22) Date of filing: 13.03.1998
(51) Int. Cl.: C07C 235/48, A61K 7/48, A61K 31/165

(54) **SALICYLIC ACID-SPHINGOID BASE DERIVATIVES AND USES THEREOF**
SALICYLSÄURE - SPHINGOIDBASE DERIVATE UND IHRE VERWENDUNG
DERIVES D'ACIDE SALICYLIQUE/BASE SPHINGOIDE ET UTILISATIONS ASSOCIEES

(30) Priority: 13.03.1997 EP 97200751
(43) Date of publication of application: 29.12.1999
(73) Proprietor: Cosmoferm B.V., 2611 XT Delft (NL)
(72) Inventor: BRAND, Ernst, Jacob, NL-1062 KM Amsterdam (NL); WEBER, Pieter, Gijsbert, NL-2985 BN Ridderkerk (NL); LAMBERS, Johannes, Wilhelmus, Jacobus, NL-2641 LB Pijnacker (NL)
(74) Representative: Gevers, Florent
(86) International application number: EP9801579
(87) International publication number: WO9840350

(56) References cited:
- EP-A- 0 662 319
- EP-A- 0 711 539
- WO-A-93/20038
- CHEMICAL ABSTRACTS, vol. 112, no. 25, 18 June 1990 Columbus, Ohio, US; abstract no. 234759n, KATSUHIKO HIGUKI ET AL: "Chemistry of succinimido esters. XVII. The erythro/threo separation of sphingolipid bases labeled with N-succinimidyl benzoates by normal-phase HPLC" page 571; XP002073190 & YUKAGAKU, vol. 38, no. 11, 1989, pages 923-928,

## Description

The present invention relates to topical application, especially of a salicylic acid derivative which is in the form of a derivative of a sphingoid base.

### Background of the invention

Salicylic acid (2-hydroxybenzoic acid) is applied in topical compositions principally for its keratinolytic properties. In addition, salicylic acid is used topically in the treatment of fungal skin infections. Cosmetic application includes the use as an antiageing agent.

Since salicylic acid is readily absorbed through the skin and excreted slowly in the urine, systemic poisoning may occur, especially after prolonged application of compositions containing high concentrations of salicylic acid.

Therefore, salicylic acid preferably is not used in one or more of the following conditions: for prolonged periods, in high concentrations, on large areas of the body, or on inflamed or broken skin.

However, it is desirable to avail of a salicylic acid formulation which is suitable for application also in the above conditions.

Now, it has been found that the use of certain salicylic acid derivatives instead of the unmodified compound enables application of salicylic acid in the above conditions.

### Description of the invention

The present invention discloses novel compounds which are formed by the coupling of the carboxyl group of salicylic acid to the amino group of a sphingoid base via an amide linkage.

The sphingoid base used for coupling of salicylic acid via an amide linkage has a general structure according to Formula 1: 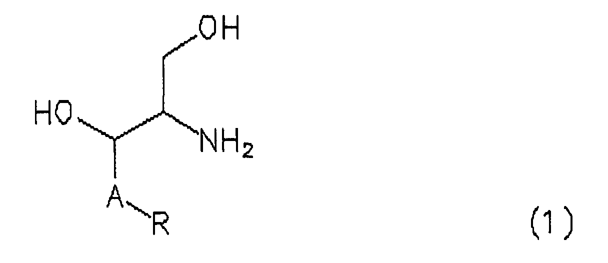 wherein:
A is CH₂-CH₂, CH=CH or C(H)OH-CH₂, and
R is a straight chain or branched alkyl group having 10 to 22 carbon atoms which may optionally contain one or more double bonds and/or may optionally be substituted with one or more hydroxyl groups.
Preferably A is C(H)OH-CH₂ and/or R is a straight chain alkyl group having 12 to 18 carbon atoms. More preferably A is C(H)OH-CH₂ and/or R is a straight chain alkyl group having 13 carbon atoms.
Optionally, one or more glycosyl moieties, or phosphate or sulphate groups, may be attached to the primary hydroxyl group.

Normally, sphingoid bases are an important constituent of ceramides. Ceramides form the largest polar lipid class of the epidermal lipids of the stratum corneum (20-40%) and are a structurally heterogenous group of sphingolipids containing the sphingoid bases sphinganine, sphingosine or phytosphingosine in amide linkage with fatty acids (both hydroxy- and non-hydroxy-fatty acids). Ceramides are supposed to have an essential role in structuring and maintaining the water impermeability barrier of the skin. Ceramides are applied in topical compositions, e.g. to maintain and/or improve the structure and flexibility of the skin.

In the salicylic acid-sphingoid base derivative the sphingoid base functions as a carrier for salicylic acid.

Due to the sphingoid base structure, the salicylic acid-sphingoid base derivative will penetrate deeper into the skin than the unmodified salicylic acid. This implies that salicylic acid as carried by the sphingoid base will be targeted to sites localised deeper within the skin, e.g. the lower layers of the stratum corneum, the mitotically active epidermal cells in the stratum granulosum and/or stratum germinativum and/or stratum spinosum, and/or the dermis. Moreover, the salicylic acid-sphingoid base derivative will penetrate into the inner layers of the skin more efficiently than salicylic acid as such.

When the salicylic acid-sphingoid base derivative according to the invention enters the stratum corneum, salicylic acid may be liberated as a result of *in situ* enzymatic hydrolysis by an esterase, e.g. a lipase, or an amidase, e.g. a ceramidase or a protease, all of which are present in the mammalian epidermis [see Wertz, P.H. and Downing, D.T., FEBS (1990), 268, 110-112; Hassler, D.F. and Bell, R.M. (1993), in "Ceramidases: Enzymology and metabolic roles", Adv. Lip. Research 26, pp.49-59 , Academic Press, San Diego]. A very advantageous aspect of the *in situ* enzymatical hydrolysis is that salicylic acid is released from the sphingoid base carrier in a controlled way and at the targeted site in the skin.

The sphingoid base thus may function as a carrier as well as a controlled release means for salicylic acid as coupled to said sphingoid base. Controlled release is particularly advantageous to ensure an even dosage of a compound which displays a very high biological activity, i.e. to minimize the chance for overdosage of said compound.

Consequently, the present invention provides the advantage that, as a result of its incorporation into a sphingoid base, much lower concentrations of salicylic acid are needed in topical compositions to reach the desired effect. Undesirable side effects of salicylic acid as coupled to a sphingoid base are thereby absent or reduced to a substantially lower level as compared to the effects of the unmodified compound.

The enzymatic hydrolysis of the compounds of the present invention which occurs in the skin will release salicylic acid as well as the sphingoid base. Sphingoid bases are also known for their biological activity. For instance, sphingoid bases can be beneficial to the skin by acting as an important physiological regulator of growth and differentiation of epidermal cells (Hannun, Y.A. and Bell, R.M., Science 1989, 243, pp. 500-507).

The present invention further provides a method for preparing salicylic acid derivatives of sphingoid bases, wherein salicylic acid is coupled to a sphingoid base via an amide linkage. Said salicylic acid-sphingoid base derivatives can be prepared by various synthesis methods known in the art. Salicylic acid can be coupled to a sphingoid base either enzymatically or chemically. Chemically, the acid can be coupled either as such using a coupling reagent, e.g. EEDQ (N-ethoxycarbonyl-2-ethoxy-1,2-dihydroquinoline), HOBT (hydroxybenzotriazole) and/or a carbodiimide, or as an activated acid e.g. a mixed anhydride (see WO93/20038) or acid halogenide.

Optionally, the salicylic acid-sphingoid base derivative may be further purified by treatment with a concentrated base to hydrolyse any diacyl compounds formed, i.e. to hydrolyze any ester bond which might be formed between the primary hydroxyl group of the sphingoid base and salicylic acid.

In a preferred embodiment of the invention, coupling of salicylic acid and a selected sphingoid base via an amide linkage occurs in the presence of HOBT and diisopropyl-carbodiimide or dicyclohexyl-carbodiimide, using tetrahydrofuran as a solvent.

The sphingoid base to which salicylic acid is coupled can be any sphingoid base according to Formula (1), including sphingoid bases selected from the group of sphingosines, 6-hydroxy-sphingosines, phytosphingosines and sphinganines, optionally with one or more glycosyl moieties, or phosphate or sulphate groups, attached to the primary hydroxyl group.

In another preferred embodiment of the invention, the sphingoid base to which salicylic acid is coupled is phytosphingosine. Phytosphingosine is obtainable efficiently by deacetylation of tetra-acetylphytosphingosine (TAPS), which in turn can be obtained in large amounts by microbial fermentation, especially by fermentation of *Pichia ciferri.* Phytosphingosine as obtained by fermentation of *Pichia ciferri* has a structure according to Formula (1), wherein A is C(H)OH-CH₂ and R is a straight chain alkyl group having 13 carbon atoms.

Another aspect of the invention relates to the use of the salicylic acid-sphingoid base derivatives of the invention in the preparation of cosmetic and/or pharmaceutical (dermatological) compositions for topical use.

Specific cosmetic and/or dermatological preparations include the usual components.

The composition comprises a vehicle to enable the active ingredient to be conveyed to the skin. Vehicles include water, solids and liquids. These are classified as emollients, emulsifiers, surfactants, solubilizers, propellants, solvents, humectants, thickeners and powders.

Emollients include alkyl higher fatty acids, natural oils, higher fatty alcohols, glyceryl and isopropyl esters, mineral oils, silicones, fatty alcohol esters.

Emulsifiers comprise compounds having a HLB (hydrophilic/lipophilic balance) value which is in the lower as well as in the higher ranges, i.e. compounds which are able to form a water-in-oil as well as compounds which are able to form an oil-in-water emulsion, respectively. Typically, if a water-in-oil emulsion is required, the HLB value of the emulsifier or mixture of emulsifiers varies between about 1 and 7. For an oil-in-water emulsion, said HLB value is higher than about 7.

Propellants include propane, butane, isobutane, dimethyl ether, chlorofluoroalkanes, carbon dioxide, nitrous oxide.

Solvents include ethyl alcohol, methylene chloride, isopropanol, ethyl ethers, DMSO, propylene glycol, butylene glycol.

Humectants include proteins and protein hydrolysates, amino acids, sorbitol, glycerin, other polyols.

Thickeners include polysaccharides, gums and carboxylic group-containing polymers

Powders including chalk, talc, starch.

The combination of the said components can account for about 5 to about 99% of the composition.

The composition containing the compounds of the invention may further include additional active ingredients, e.g. ingredients which may increase the efficacy of the salicylic acid-sphingoid base derivative in a certain application.

The compositions containing the compounds of the invention are suitable for topical use. The amount of salicylic acid-sphingoid base derivative as present in a composition suitable for topical application ranges from 0.0001% to 25%, preferably from 0.001% to 5%, more preferably from 0.005 to 2%, most preferably from 0.01% to 1% by weight of the composition.

The compositions containing the salicylic acid-sphingoid base derivatives of the invention may be used in any of the fields where topical application of salicylic acid is desired. For instance, the compositions of the invention are advantageously used in the treatment of hyperkeratotic and scaling skin conditions, such as psoriasis, ichtiosis, seborrhoeic dermatitis, atopic dermatitis, acne, and the like.

The compositions of the invention further are particularly useful in those applications wherein salicylic acid may act synergistically with a spingoid base, e.g. in applications wherein an antimicrobial, especially an antifungal, or an antiinflammatory efficacy is desired.

### Example 1

### Synthesis of N-salicyloyl-phytosohingosine

A mixture of 50 grams of phytosphingosine, 25 grams of salicylic acid (Acros), 430 ml of tetrahydrofuran (Merck) and 22.5 grams of 1-hydroxybenzotriazol (Acros) was stirred under nitrogen. All solids were rinsed into the flask with 50 ml of tetrahydrofuran. The temperature was 15°C. Next 30 ml of diisopropyl-carbodiimide (Sigma) was added over a period of 14 minutes causing the temperature to go up to 31°C. After stirring at 35°C for 1.5 hours 25 ml of water was added (suspension => clear solution) and the mixture was distilled off until the liquid temperature reached 80°C (440 ml distillate).

After cooling 500 ml of methanol was added followed by 125 ml of water and the mixture was cooled to 0°C in 1 hour while stirring gently. After stirring at 0°C for 1 hour the mixture was filtered off with suction. The filtercake was washed with a cold (5°C) mixture of 250 ml of methanol and 100 ml of water to give 270 g of cake.

The cake was heated with 450 ml of methanol to 63°C. The pH was adjusted from 3.7 to 10.7 with 17.3 grams of 50% w/w NaOH. After stirring for 1.5 hour at ca 63°C (pH=10.7) the pH was adjusted with acetic acid to 6.7 ( 15 g). The mixture was filtered while hot and the residue was washed with 50 ml of methanol. To the filtrate 200 ml of methanol and 100 ml of water were successively added, the mixture was cooled while stirring and crystallisation started at ca. 27°C. The mixture was further cooled to 0°C in about 1 hour. After stirring at 0°C for 1 hour the precipitate was filtered off and washed with a cold mixture of 300 ml of methanol and 100 ml of water. The wet filtercake weighted 195 grams. The wet cake was dried overnight at 35°C (ventilated) and under vacuum at 40°C to give 54 grams of N-salicyloyl-phytosphingosine.

## Claims

1. A compound which is a salicylic acid-sphingoid base derivative wherein salicylic acid is coupled to a sphingoid base having a general structure according to Formula 1 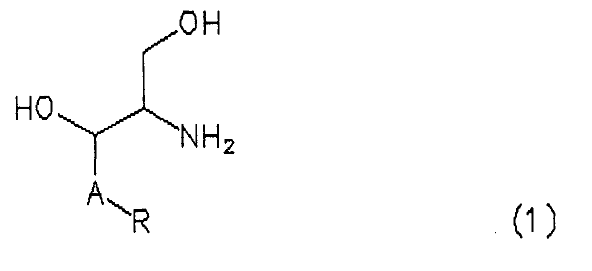 wherein:
A is CH₂-CH₂, CH=CH or C(H)OH-CH₂,
R is a straight chain or branched alkyl group having 10 to 22 carbon atoms which may optionally contain one or more double bonds and/or may optionally be substituted with one or more hydroxyl groups, and
optionally, one or more glycosyl moieties, or phosphate or sulphate groups, may be attached to the primary hydroxyl group.
via an amide linkage.

2. The compound of claim 1, wherein A is C(H)OH-CH₂.

3. The compound of claims 1 or 2, wherein R is a straight chain alkyl group having 12 to 18 carbon atoms, preferably a straight chain alkyl group having 13 carbon atoms.

4. A process for the preparation of the compounds of any one of the claims 1 to 3, wherein salicylic acid is coupled to the sphingoid base either as such, using a coupling reagent, e.g. EEDQ (N-ethoxycarbonyl-2-ethoxy-1,2-dihydroquinoline), HOBT (hydroxybenzotriazole) and/or a carbodiimide, or as an activated acid e.g. a mixed anhydride or acid halogenide.

5. The process of claim 4, wherein salicylic acid is coupled to the sphingoid base using a coupling reagent, e.g. EEDQ (N-ethoxycarbonyl-2-ethoxy-1,2-dihydroquinoline), HOBT (hydroxybenzotriazole) and/or a carbodiimide.

6. The process of claim 5, wherein the coupling reagent is HOBT and diisopropyl-carbodiimide or dicyclohexylcarbodiimide.

7. A cosmetic composition comprising the compound of any one of the claims 1 to 3.

8. A dermatological composition comprising the compound of any one of the claims 1 to 3.

9. The composition of claims 7 or 8, wherein the salicylic acid-sphingoid base derivative is present in a concentration varying from 0.0001% to 25%, preferably from 0.001% to 5%, more preferably from 0.005 to 2%, most preferably from 0.01% to 1% by weight of the composition.

10. The compound of any one of the claims 1 to 3 for use as a pharmaceutical.

11. Use of the compound of any one of the claims 1 to 3 as a cosmetic.

## Patentansprüche

1. Verbindungen, bei denen es sich um Salicylsäure-Sphingoidbase-Derivate handelt, wobei Salicylsäure über einer Amidbindung mit einer Sphingoidbase mit einer allgemeinen Struktur gemäß Formel 1 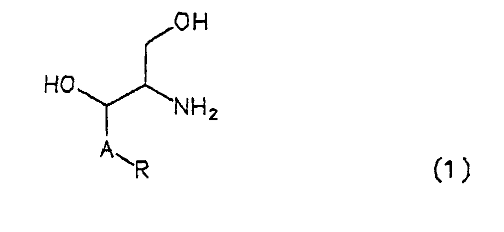 gekuppelt ist, wobei:
A für CH₂-CH₂, CH=CH oder C(H)OH-CH₂ steht,
R für eine geradkettige oder verzweigte Alkylgruppe mit 10 bis 22 Kohlenstoffatomen, die gegebenenfalls ein oder mehrere Doppelbindungen enthalten kann und/oder gegebenenfalls durch eine oder mehrere Hydroxylgruppen substituiert sein kann, steht, und
gegebenenfalls eine oder mehrere Glykosyleinheiten oder Phosphat- oder Sulfatgruppen an die primäre Hydroxylgruppe gebunden sein können.

2. Verbindungen nach Anspruch 1, wobei A für C(H)OH-CH₂ steht.

3. Verbindungen nach Anspruch 1 oder 2, wobei R für eine geradkettige Alkylgruppe mit 12 bis 18 Kohlenstoffatomen, vorzugsweise eine geradkettige Alkylgruppe mit 13 Kohlenstoffatomen, steht.

4. Verfahren zur Herstellung der Verbindungen nach einem der Ansprüche 1 bis 3, bei dem man Salicylsäure entweder als solche unter Verwendung eines Kupplungsmittels, z.B. EEDC (N-Ethoxycarbonyl-2-ethoxy-1,2-dihydrochinolin), HOBT (Hydroxybenzotriazol) und/oder eines Carbodiimids oder als aktivierte Säure, z.B. als gemischtes Anhydrid oder Säurehalogenid, mit der Sphingoidbase kuppelt.

5. Verfahren nach Anspruch 4, bei dem man die Salicylsäure unter Verwendung eines Kupplungsmittels, z.B. EEDC (N-Ethoxycarbonyl-2-ethoxy-1,2-dihydrochinolin), HOBT (Hydroxybenzotriazol) und/oder eines Carbodiimids an die Sphingoidbase kuppelt.

6. Verfahren nach Anspruch 5, wobei es sich bei dem Kupplungsmittel um HOBT und Diisopropylcarbodiimid oder Dicyclohexylcarbodiimid handelt.

7. Kosmetische Zusammensetzung, enthaltend eine Verbindung nach einem der Ansprüche 1 bis 3.

8. Dermatologische Zusammensetzung, enthaltend eine Verbindung nach einem der Ansprüche 1 bis 3.

9. Zusammensetzung nach Anspruch 7 oder 8, in der das Salicylsäure-Sphingoidbase-Derivat in einer Konzentration von 0,0001% bis 25%, vorzugsweise von 0,001% bis 5%, besonders bevorzugt von 0,005 bis 2%, ganz besonders bevorzugt von 0,01% bis 1%, des Gewichtes der Zusammensetzung vorliegt.

10. Verbindungen nach einem der Ansprüche 1 bis 3 zur Verwendung als Arzneimittel.

11. Verwendung einer Verbindung nach einem der Ansprüche 1 bis 3 als Kosmetikum.

## Revendications

1. Composé qui est un dérivé d'acide salicylique-base sphingoïde, **caractérisé en ce que** l'acide salicylique est couplé à une base sphingoïde ayant une structure générale selon la formule 1 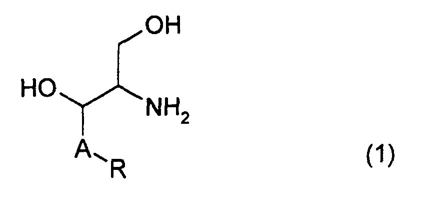 dans laquelle:
A est CH₂-CH₂, CH=CH ou C(H)OH-CH₂,
R est un groupement alkyle à chaîne linéaire ou ramifiée ayant de 10 à 22 atomes de carbone, pouvant éventuellement contenir une ou plusieurs doubles liaisons et/ou pouvant éventuellement être substitué par un ou plusieurs groupements hydroxy, et
éventuellement, un ou plusieurs motifs glycosyle, ou groupements phosphate ou sulfate, peuvent être liés au groupement hydroxy primaire
par l'intermédiaire d'une liaison amide.

2. Composé selon la revendication 1, **caractérisé en ce que** A est C(H)OH-CH₂.

3. Composé selon la revendication 1 ou 2, **caractérisé en ce que** R est un groupement alkyle à chaîne linéaire ayant de 12 à 18 atomes de carbone, de préférence un groupement alkyle à chaîne linéaire ayant 13 atomes de carbone.

4. Procédé de préparation de composés selon l'une quelconque des revendications 1 à 3, **caractérisé en ce que** l'acide salicylique est couplé à la base sphingoïde soit telle quelle, en utilisant un agent de couplage, par ex. l'EEDQ (la N-éthoxycarbonyl-2-éthoxy-1,2-dihydroquinoléine), l'HOBT (l'hydroxybenzotriazole) et/ou un carbodiimide, soit sous forme d'acide activé, par ex. un anhydre mixte ou un halogénure d'acide.

5. Procédé selon la revendication 4, **caractérisé en ce que** l'acide salicylique est couplé à la base sphingoïde en utilisant un réactif de couplage, par ex. l'EEDQ (la N-éthoxycarbonyl-2-éthoxy-1,2-dihydroquinoléine), l'HOBT (l'hydroxybenzotriazole) et/ou un carbodiimide.

6. Procédé selon la revendication 5, **caractérisé en ce que** l'agent de couplage est l'HOTB et le diisopropyl-carbodiimide ou le dicyclohexylcarbodiimide.

7. Composition cosmétique comprenant le composé selon l'une quelconque des revendications 1 à 3.

8. Composition dermatologique comprenant le composé selon l'une quelconque des revendications 1 à 3.

9. Composition selon les revendications 7 ou 8, **caractérisée en ce que** le dérivé d'acide salicylique-base sphingoïde est présent dans une concentration allant de 0,0001% à 25%, de préférence de 0,001% à 5%, plus préférablement de 0,005 à 2%, de manière tout à fait préférée de 0,01% à 1% en poids de la composition.

10. Composé selon l'une quelconque des revendications 1 à 3 destiné à être utilisé en pharmacie.

11. Utilisation du composé selon l'une quelconque des revendications 1 à 3 en cosmétique.
